Europäisches Patentamt

European Patent Office

Office européen des brevets

(19)

(11) **EP 0 667 356 B1**

(12) **FASCICULE DE BREVET EUROPEEN**

(45) Date de publication et mention
de la délivrance du brevet:
**01.07.1998 Bulletin 1998/27**

(51) Int. Cl.$^6$: **C08B 30/18**, C12P 19/14,
A61M 1/28

(21) Numéro de dépôt: **95400292.9**

(22) Date de dépôt: **13.02.1995**

(54) **Procédé de fabrication d'un hydrolysat d'amidon à faible indice de polymolécularité, hydrolysat d'amidon ainsi obtenu et son utilisation en dialyse péritonéale voir au-dessus**

Verfahren zur Herstellung eines Stärkehydrolysats mit niedrigem Polymolekularitätindex; so hergestelltes Stärkehydrolysat und seine Anwendung in der Peritonealdialyse

Process for preparing a starch hydrolysate with low polymolecularity index, starch hydrolysate so obtained and its use in peritoneal dialysis

(84) Etats contractants désignés:
**AT BE CH DE DK ES FR GB IT LI NL SE**

(30) Priorité: **15.02.1994 FR 9401707**

(43) Date de publication de la demande:
**16.08.1995 Bulletin 1995/33**

(73) Titulaire: **Roquette Frères**
**62136 Lestrem (FR)**

(72) Inventeurs:
- **Fouache née Ducroquet, Catherine**
  **F-62113 Sailly Labourse (FR)**
- **Duflot, Pierrick**
  **F-62136 Richebourg (FR)**

(74) Mandataire:
**Boulinguiez, Didier et al**
**Cabinet Plasseraud**
**84, rue d'Amsterdam**
**75440 Paris Cedex 09 (FR)**

(56) Documents cités:
EP-A- 0 115 911          EP-A- 0 207 676
WO-A-93/07177          FR-A- 1 548 941
US-A- 3 974 032

- **STARKE., vol. 35, WEINHEIM DE, page 98 E.**
  **CORÜSLÜ ET AL. 'Starch hydrolysates with very**
  **low degrees of polydispersity'**

## Description

La présente invention se rapporte à un procédé de fabrication d'un hydrolysat d'amidon à faible indice de polymolécularité.

De façon plus précise elle se rapporte à un procédé de fabrication d'un hydrolysat d'amidon à faible indice de polymolécularité particulièrement adapté à la technique de la dialyse péritonéale continue et ambulatoire. Elle vise également, à titre de produit nouveau, l'hydrolysat d'amidon ainsi obtenu, ainsi que l'utilisation dudit hydrolysat comme agent osmotique en dialyse péritonéale ambulatoire continue.

Les hydrolysats d'amidon standards sont produits par hydrolyse acide ou enzymatique d'amidon de céréales ou de tubercules. Ils sont en fait un mélange de glucose et de polymères du glucose, de poids moléculaires extrêmement variés.

Une première façon de les classifier est la mesure de leur pouvoir réducteur, exprimée classiquement par la notion de dextrose équivalent ou D.E. Par définition, on fixe au dextrose ou glucose pur, monomère constitutif de ces polymères, un D.E. de 100. L'amidon, très haut polymère du glucose, possède un D.E. proche de 0. Entre ces deux valeurs on trouve toute une gamme d'hydrolysats d'amidon, les plus hydrolysés ayant un D.E. proche de 100, les moins hydrolysés ayant un D.E. tendant vers 0.

Une telle mesure du D.E. est cependant insuffisante pour représenter précisément la composition moléculaire de l'hydrolysat d'amidon. Ainsi par exemple le maltose, polymère de glucose formé de deux molécules de ce sucre, de degré de polymérisation (D.P.) égal à 2 et qui ne possède qu'une extrémité réductrice pour deux molécules de glucose, montre un D.E. théorique voisin de 50, qui est le même que celui fourni par un mélange à parts égales en poids d'amidon et de glucose.

L'hydrolyse acide de l'amidon, totalement aléatoire, ou son hydrolyse enzymatique un peu plus ordonnée, fournissent des mélanges de glucose et de polymères du glucose que la seule mesure du D.E ne permet donc pas de définir avec précision et qui comportent des molécules très courtes, de faible D.P., aussi bien que des molécules très longues, de D.P. élevé.

La mesure du D.E. ne donne en fait qu'une idée approximative du degré de polymérisation moyen de ces polymères et donc de leur masse moléculaire moyenne en nombre : Mn

Cette masse moléculaire moyenne en nombre Mn est en fait exprimée par l'équation :

$$M_n = \frac{1}{\Sigma \dfrac{\text{fraction en poids de chaque molécule}}{\text{masse moléculaire de cette molécule}}}$$

Dans l'exemple du mélange à parts égales en poids d'amidon et de glucose, on obtient donc :

$$M_n = \frac{1}{\dfrac{0,5}{180} + \dfrac{0,5}{\infty}} \text{ soit } \frac{180}{0,5} = 360,$$

ce qui correspond à deux fois le poids moléculaire du glucose, soit approximativement le poids moléculaire du maltose.

Tout comme le D.E., Mn ne permet donc pas de caractériser totalement la dispersion des masses moléculaires des mélanges de polymères que sont les hydrolysats d'amidon puisqu'il ne permet pas non plus d'effectuer la différence entre le maltose et le mélange à parts égales d'amidon et de glucose.

On définit alors, pour pouvoir appréhender cette différence, la masse moléculaire moyenne en poids : Mp, qui est elle exprimée par l'équation :

$$Mp = \Sigma \text{ fraction en poids de chaque molécule x masse moléculaire de cette molécule.}$$

Dans l'exemple qui nous intéresse, on obtient donc :

Mp = 0,5 x 180 + 0,5 x $\infty$ = $\infty$ , résultat qui est fort éloigné du poids moléculaire du maltose, qui est égal à 342, mais qui reflète bien cette fois l'existence d'un haut polymère dans le mélange.

Par souci de simplification, on a admis dans ce qui précède que l'amidon possède une masse moléculaire infinie. Bien qu'il s'agisse d'un très haut polymère du glucose, sa masse moléculaire est en réalité finie et se situe à quelques millions de daltons. De même, on n'a pas tenu compte de l'eau réactionnelle nécessaire à l'hydrolyse totale du polymère mais qui ne représente que 10 % du poids de celui-ci et même seulement 5 % du poids du maltose.

Le rapport Mp/Mn est appelé souvent "indice de polymolécularité" et permet de caractériser globalement la dispersité des masses moléculaires d'un mélange polymérique.

Dans le cas idéal du maltose pur, ce rapport est de 1, il tendrait vers l'infini dans le cas du mélange à parts égales d'amidon et de glucose.

Dans la pratique, les valeurs de Mn et Mp permettant de mieux définir les espèces polymoléculaires composant les mélanges de polymères ne se calculent pas mais se mesurent par différentes techniques. L'osmométrie, l'ébulliométrie, la cryométrie, la distillation isotherme, la méthode thermoélectrique ou le dosage chimique des groupements terminaux des polymères, comme le D.E. permettent l'accès au Mn. La diffusion de la lumière permet l'accès au Mp et parfois au Mn. Ces deux valeurs, Mp et Mn, peuvent aussi être accessibles par chromatographie de perméation de gel, sur des colonnes de chromatographie étalonnées avec des dextrans de masses moléculaires connues (Alsop et Al., Process Biochem, 12, 15-22; 1977 ou Alsop et Al., J. Chromatography 246, 227-240; 1982). Cette dernière méthode de mesure est très adaptée aux polymères de glucose et c'est celle qui est utilisée dans le cadre de la présente invention.

La demande de brevet britannique GB 2.247.242 décrit des granules d'amylose qui précipitent en solution aqueuse, qui possèdent un Mn de 4000 à 7000 daltons et un indice de polydispersité particulièrement bas, de 1,4 à 1,7, fixant, pour ces granules, un Mp de 5.600 à 11.900 daltons.

La demande de brevet européen n° 207.676 enseigne que pour un usage en dialyse péritonéale continue et ambulatoire, des hydrolysats d'amidon formant des solutions limpides et incolores à 10 % dans l'eau et ayant un poids moléculaire moyen en poids (Mp) de 5.000 à 100.000 daltons et un poids moléculaire moyen en nombre (Mn) inférieur à 8.000 daltons sont préférés.

De tels hydrolysats d'amidon comprennent aussi de façon préférée au moins 80 % de polymères du glucose dont le poids moléculaire est compris entre 5.000 et 50.000 daltons, peu ou pas de glucose ou de polymères du glucose de DP inférieur ou égal à 3 (poids moléculaire 504) et peu ou pas de polymères de glucose de poids moléculaire supérieur à 100.000 (DP voisin de 600).

En d'autres termes, les hydrolysats d'amidon préférés sont des hydrolysats d'amidon de faible indice de polymolécularité.

On conçoit en effet aisément pour cette application que les monomères ou polymères de faible poids moléculaire traversent rapidement la paroi péritonéale et sont ainsi sans intérêt durable pour la création d'un gradient de pression osmotique, et que les polymères de très haut poids moléculaire, dénués de pouvoir osmotique, sont à éviter et même à proscrire puisque potentiellement dangereux s'il leur advenait de précipiter consécutivement à leur rétrogradation.

Les procédés proposés dans cette demande de brevet pour obtenir ces hydrolysats d'amidon de faible indice de polymolécularité consistent :

- soit à effectuer une précipitation fractionnée d'une maltodextrine à l'aide d'un solvant miscible à l'eau.
- soit à effectuer une filtration moléculaire de cette même maltodextrine au travers de différentes membranes possédant un seuil de coupure ou d'exclusion adéquat.

Dans les deux cas, ces procédés visent à éliminer à la fois les polymères de très haut poids moléculaire et les monomères ou oligomères de faible poids moléculaire.

Ces procédés ne donnent toutefois pas satisfaction tant du point de vue de leur mise en oeuvre que du point de vue des rendements et de la qualité des produits qu'ils permettent d'obtenir.

Ils sont en effet très délicats à mettre en oeuvre car ils nécessitent de manipuler des volumes d'eau énormes ou, pire, de solvants organiques coûteux et dangereux. Leur rendement est faible puisqu'ils ne permettent pas d'obtenir plus de 25% d'hydrolysat d'amidon de faible indice de polymolécularité par rapport à l'hydrolysat d'amidon standard mis en oeuvre. Ces procédés ne permettent pas non plus d'obtenir des hydrolysats d'amidon dont l'indice de polymolécularité est particulièrement faible, ce qui est cependant souhaitable pour les raisons déjà exposées plus haut. Dans la demande de brevet européen n° 207 676, les indices de polymolécularité des produits obtenus s'échelonnent en effet de 7 à 2,85 dans le meilleur des cas.

Soucieuse de mettre au point un procédé de fabrication d'un hydrolysat d'amidon complètement soluble dans l'eau et de faible indice de polymolécularité, inférieur à 2,8 et de préférence inférieur à 2,5, ayant de préférence un Mn inférieur à 8.000 daltons et possédant un Mp compris entre 5.000 et 100.000 daltons, de préférence compris entre 8.000 et 50.000 daltons et plus préférentiellement encore compris entre 12.000 et 20.000 daltons, procédé qui soit dépourvu des inconvénients de l'art antérieur, la société Demanderesse, qui s'est attachée à résoudre ce problème, a constaté au terme de multiples essais qu'un tel hydrolysat d'amidon pouvait être obtenu par la mise en oeuvre d'un procédé consistant à :

- hydrolyser par voie acide un lait d'amidon waxy jusqu'à un D.E. compris entre 8 et 15 ;
- éventuellement compléter cette hydrolyse acide par une hydrolyse enzymatique à l'aide d'alpha-amylase bactérienne jusqu'à un D.E. compris entre 11 et 18 ;

- chromatographier sur résines cationiques fortes macroporeuses sous forme alcaline ou alcalino-terreuse cet hydrolysat double acide-enzyme ;
- collecter l'hydrolysat d'amidon exclu lors de cette étape de chromatographie.

On préfère collecter l'hydrolysat d'amidon exclu lors de cette étape de chromatographie dans un rendement pondéral généralement compris entre 10 et 80 % de l'hydrolysat mis en oeuvre à l'étape de chromatographie.

Plus on abaisse le rendement pondéral de la chromatographie, plus on abaisse l'indice de polydispersité. Des rendements de 50 % sont ordinairement traduits par des indices de polydispersité inférieurs à 2,5.

Le procédé selon l'invention permet d'obtenir des hydrolysats d'amidon complètement solubles dans l'eau et d'indice de polymolécularité particulièrement faible. L'invention vise donc, à titre de produit industriel nouveau, un hydrolysat d'amidon cireux caractérisé par le fait qu'il présente un indice de polymolécularité inférieur à 2,8 et de préférence inférieur à 2,5, une masse moléculaire moyenne en poids Mp comprise entre 8000 et 22.500 daltons, et plus préférentiellement encore comprise entre 12.000 et 20.000 daltons, ainsi qu'une masse moléculaire moyenne en nombre Mn inférieure à 8.000 daltons.

Cet hydrolysat d'amidon en question contient de préférence moins de 3 % de glucose et de polymères de glucose de DP inférieur ou égal à 3 et moins de 0,5 % de polymères de glucose de DP supérieur à 600.

La société Demanderesse a découvert que seuls les amidons composés presque exclusivement d'amylopectine et couramment dénommés amidons waxy ou amidons cireux pouvaient servir de matière première dans le procédé de l'invention. Les amidons ou fécules contenant une proportion non négligeable d'amylose ne conviennent pas.

De préférence, l'amidon mis en oeuvre dans le procédé de l'invention est un amidon de maïs cireux mais les amidons de riz waxy ou les fécules waxy peuvent convenir également.

De préférence l'hydrolyse acide est effectuée à l'aide d'acide chlorhydrique en quantité suffisante pour porter l'acidité du lait d'amidon à une valeur comprise entre 20 et 50 meq/l (milliéquivalents par litre). La température à laquelle se déroule l'hydrolyse peut être comprise entre 105 et 135°C, mais on préfère généralement travailler sous pression à une température voisine de 117°C. La durée d'hydrolyse est variable et est inversement corrélée à la quantité d'acide mise en oeuvre et à la température à laquelle on effectue celle-ci.

L'interdépendance de ces trois paramètres est bien connue de l'homme de l'art qui sait les ajuster pour qu'au terme de cette étape soit obtenu un hydrolysat d'amidon dont le D.E. est compris entre 8 et 15. Des valeurs de D.E. s'écartant trop de ces valeurs ne permettraient pas d'obtenir avec de bons rendements les hydrolysats d'amidon à faible polymolécularité.

L'éventuelle étape d'hydrolyse enzymatique à l'aide d'alpha-amylase bactérienne, est effectuée de préférence à une température comprise entre 40 et 100°C et de préférence entre 50 et 80°C. Une telle étape qui se traduit surtout par l'hydrolyse des produits de très haut poids moléculaire permet, à rendement de chromatographie égal, d'abaisser l'indice de polydispersité des produits obtenus.

Le pH du milieu réactionnel est alors réglé au moyen de chaux par exemple à une valeur proche du pH optimal de l'alpha-amylase employée et qui est généralement compris entre 5 et 7.

La quantité d'enzyme employée est de préférence comprise entre 200 et 2.000 unités TAU (Thermostable amylase units) par kilogramme d'amidon mis en oeuvre. Cette unité non conventionnelle est définie ainsi : une unité TAU est la quantité d'enzyme qui convertit dans les conditions standard 1 mg d'amidon soluble par minute en un produit ayant le même pouvoir d'absorption à 620 nm, après réaction à l'iode, qu'une couleur de référence. Ces conditions standard sont : pH 6,6, température 30°C, durée de la réaction de 15 à 25 minutes.

Il est possible d'obtenir le protocole précis de cette mesure auprès de la société GIST.

On préfère toutefois mettre en oeuvre une quantité d'enzyme comprise entre 500 et 1500 TAU par kilogramme d'amidon, quantité qui est telle qu'elle ne soit pas trop faible pour que la réaction se déroule en un temps acceptable et qui est telle qu'elle ne soit pas trop forte de manière à pouvoir arrêter à temps cette hydrolyse dont on rappelle qu'il convient d'y mettre un terme à l'obtention d'un D.E. compris entre 11 et 18, de préférence entre 12 et 15.

Encore une fois, des valeurs de D.E. s'écartant trop de ces valeurs ne permettraient pas d'obtenir avec de bons rendements les hydrolysats d'amidon à faible polymolécularité qui sont visés comme produits du procédé selon l'invention.

Pour arrêter cette hydrolyse, on peut utiliser tout procédé de dénaturation chimique ou physique de l'alpha-amylase. On préfère cependant utiliser un procédé physico-chimique, et celui-ci est commodément réalisé par chauffage du milieu réactionnel à une température de 70 à 120°C durant 5 à 60 minutes, après avoir porté ledit milieu à un pH inférieur à 4.

Il est préférable, avant de procéder à l'étape suivante de la chromatographie du milieu réactionnel obtenu par hydrolyse acide puis éventuellement enzymatique de l'amidon waxy, d'effectuer les opérations classiques de filtration et de déminéralisation, qui ont pour but d'éliminer les protéines insolubles ou insolubilisées par le traitement physique ou chimique de dénaturation enzymatique, ainsi que les sels minéraux ou autres impuretés ioniques ou non chargées contenues dans l'amidon mis en oeuvre ou ajoutées pour réaliser les traitements précédents.

L'étape de chromatographie s'effectue de manière connue en soi en relation avec d'autres hydrolysats d'amidon. Elle peut être effectuée de façon discontinue ou continue (lit mobile simulé), mais doit être effectuée sur des adsorbants du type résines cationiques macroporeuses car la société Demanderesse a découvert que les résines cationiques de structure gel, employées traditionnellement dans le fractionnement des hydrolysats d'amidon ou de leurs isomérisats ne convenaient pas pour cette application. Il faut également que ces résines soient des résines cationiques fortes et qu'elles soient mises sous forme alcaline ou éventuellement alcalino-terreuse.

La forme potassium K+, est cependant préférée.

Cette étape de chromatographie peut être réalisée par exemple en employant le procédé et l'appareillage décrit dans le brevet américain US 4 422 881 dont la société Demanderesse est cessionnaire. Il est préférable également que les résines employées au titre d'adsorbant chromatographique aient une granulométrie homogène et comprise entre 100 et 1.500 microns environ. Une granulométrie préférée est de 200 à 500 microns. Un type de résines particulièrement apprécié pour la mise en oeuvre de ce procédé est la résine C 150 sous forme K+, commercialisée par la société PUROLITE.

Le choix des paramètres du fonctionnement chromatographique, parmi lesquels on note plus particulièrement les débits relatifs d'élution, d'alimentation en sirop à fractionner, c'est à dire dans ce cas d'hydrolysat d'amidon waxy acide puis éventuellement enzymatique, le débit d'extraction de la fraction constituant l'hydrolysat d'amidon à faible indice de polymolécularité et le débit relatif de la fraction rassemblant essentiellement les polymères de glucose de faible poids moléculaire retenus par la résine et enfin la composition des zones de désorption, d'adsorption et d'enrichissement lorsqu'on utilise le procédé et appareillage du brevet américain US 4.422.881 est expliqué et illustré dans l'exemple.

Le choix de ces paramètres est effectué de manière telle que la fraction $X_1$ constituant l'hydrolysat d'amidon à faible indice de polymolécularité représente de 10 à 80 %, de préférence de 30 à 70 % et encore plus préférentiellement de 40 à 60 % de la matière sèche de l'hydrolysat d'amidon waxy acide-enzyme mis en oeuvre dans cette étape de fractionnement.

Pour parvenir à ce résultat, ces paramètres sont choisis comme suit quand le fractionnement chromatographique est réalisé selon les enseignements du brevet américain précité et quand l'adsorbant utilisé est une résine cationique macroporeuse réticulée à environ 7,5 % de divinylbenzène, d'une granulométrie comprise entre 400 et 1200 microns, et quand cette résine est utilisée sous forme $K^+$ :

- débit d'élution relatif de 75 à 750 l/h/m$^3$ d'adsorbant.
- débit d'alimentation relatif en hydrolysat d'amidon waxy, de 10 à 100 l/h/m$^3$ d'adsorbant
- débit d'extraction relatif de la fraction $X_1$ constituant l'hydrolysat d'amidon à faible polymolécularité, de 65 à 650 l/h/m$^3$ d'adsorbant.
- débit d'extraction relatif de la fraction $X_2$ contenant essentiellement les polymères de glucose de faible poids moléculaire, de 20 à 200 l/h/m$^3$ d'adsorbant.

L'hydrolyse de l'amidon cireux mis en oeuvre dans les conditions particulières du procédé de la présente invention fait que les hydrolysats mis en oeuvre à l'étape de chromatographie ne contiennent pas de façon décelable de polymères du glucose dont la masse moléculaire est supérieure à 200 000 daltons. Ceci simplifie énormément cette étape de chromatographie puisqu'il n'est pas nécessaire de fractionner à nouveau la fraction $X_1$ pour n'en conserver que les polymères du glucose de taille moyenne. Cet état de fait explique en partie l'excellent rendement en hydrolysat d'amidon à faible polymolécularité que permet d'obtenir le procédé de l'invention.

La fraction $X_1$ peut en l'état et après purifications traditionnelles par décoloration, filtration, déminéralisation, servir de base à la confection des solutions de dialyse puisqu'elle ne contient pas de matières insolubles, mais elle peut aussi être avantageusement modifiée par voie chimique ou biologique pour accroître sa stabilité, notamment thermique.

C'est ainsi que cette fraction peut être hydrogénée pour fournir des polymères dont l'extrémité finale glucose réductrice est remplacée par une extrémité sorbitol non réductrice.

Une telle transformation peut être obtenue notamment par des techniques d'hydrogénation catalytique bien connues de l'homme de l'art. Ces techniques consistent par exemple à soumettre une solution d'un sucre réducteur, en l'occurrence l'hydrolysat d'amidon à faible indice de polymécularité, en présence d'un catalyseur au nickel de Raney, à une pression d'hydrogène de 40 à 70 kg/cm$^2$ et à une température de 100 à 150°C. L'hydrogénation est poursuivie quelques heures jusqu'à ce que le produit hydrogéné ne montre pratiquement plus de pouvoir réducteur.

Cette fraction $X_1$ peut également être soumise à l'action d'enzymes extraites notamment de bactéries du genre Rhizobium ou Arthrobacter qui ont la particularité d'isomériser le chaînon maltose réducteur terminal des polymères obtenus par le procédé de l'invention en chaînon alpha-alpha tréhalose non réducteur.

Une telle isomérisation peut être obtenue par exemple grâce aux techniques dévoilées dans la demande de brevet européen EP 606 753 dont les enseignements sont incorporés ici par référence. Elle consiste à soumettre une solution de l'hydrolysat d'amidon à faible indice de polymolécularité à l'action d'une enzyme formant des saccharides non réducteurs à une température de 40 à 55°C et un pH compris entre 5 et 10. La quasi-absence de molécules de faible poids moléculaire (glucose, maltose, maltotriose...) dans les produits obtenus par le procédé de la présente invention, produits sur lesquels l'enzyme est peu active, permet d'obtenir des produits qui ne montrent que très peu de pouvoir réducteur et qui malgré tout, ne sont composés d'unités anhydroglucose.

Il faut noter ici qu'aucun de ces deux procédés, hydrogénation ou isomérisation enzymatique ne modifie sensiblement le Mp, le Mn ou l'indice de polymécularité des produits soumis à ces actions. De même, la solubilité dans l'eau de ces produits demeure totale.

Bien qu'ils soient particulièrement bien adaptés comme agents osmotiques utilisables en dialyse péritonéale, les produits de l'invention, qu'il s'agisse des hydrolysats d'amidon de faible polymécularité ou de leurs équivalents hydrogénés ou isomérisés sont également utilisables en tant que supports très peu hygroscopiques d'édulcorants intenses, de colorants, de parfums, de café ou thé solubles et peuvent être utilisés avec avantage dans tous les cas d'utilisation des maltodextrines traditionnelles de bas DE, insensibles à la rétrogradation.

La fraction $X_2$, composée de polymères du glucose de faible poids moléculaire peut en outre être récupérée dans son intégralité et être hydrolysée complètement en glucose par exemple, ce qui contribue encore à l'économie du procédé de l'invention.

Cette fraction $X_2$ peut aussi être commercialisée en l'état comme une maltodextrine de D.E. voisin de 22, exempte de polymères relativement lourds du glucose.

L'exemple qui suit a pour objet d'illustrer l'invention. Il ne saurait être limitatif en particulier pour ce qui constitue l'étape de chromatographie, qui n'est ici décrite que pour un matériel particulier permettant une mise en oeuvre particulière d'un procédé continu.

Des procédés discontinus faisant appel à une ou plusieurs colonnes peuvent aussi être utilisés dans le procédé de l'invention, de même que d'autres systèmes continus. La géométrie des dispositifs employés et l'efficacité des mouvements pistons générés au coeur des résines peuvent avoir une influence sur les paramètres ci-dessus évoqués mais l'homme de l'art saura rapidement trouver dans quel sens il convient de les modifier autour des valeurs moyennes indiquées s'il ne dispose pas exactement de l'installation ici décrite.

EXEMPLE

Liquéfaction acide

On procède à la liquéfaction par voie acide d'un lait d'amidon de waxy maïs d'une matière sèche de 320 g/kg à la température de 117°C. On utilise pour ce faire de l'acide chlorhydrique que l'on ajoute au lait d'amidon de sorte que celui-ci présente une acidité de 34 meg/l.

On met un terme à cette liquéfaction par voie acide en refroidissant l'hydrolysat dès que celui-ci atteint le DE de 11,5.

On peut alors mesurer pour cet hydrolysat un Mp de 10.070 et un Mn de 2.020. La teneur en polymères de poids moléculaire supérieur à 100.000 daltons est d'environ 0.18 %, celle de DP1, DP2, DP3 est d'environ 7 %.

Liquéfaction enzymatique

L'hydrolysat acide ainsi obtenu est ajusté au pH de 6,2, sa température est amenée à 65°C puis on y ajoute 0,15 ‰ en poids d'alpha amylase bactérienne MAXAMYL[R] commercialisée par la société GIST. Cette enzyme possède une activité spécifique de 6500 TAU/g. On laisse l'hydrolyse se poursuivre jusqu'à l'obtention d'un DE de 12,6, puis on porte rapidement le tout cinq minutes à 120°C pour dénaturer l'enzyme.

On peut alors mesurer pour cet hydrolysat double, acide-enzyme, un Mp de 8600 et un Mn de 1650. La teneur en polymères de poids moléculaire supérieur à 100.000 daltons n'est plus que de 0,1 % environ. La teneur en DP1 + DP2 + DP3 est d'environ 8,5 %. Cet hydrolysat est alors filtré, décoloré, déminéralisé comme il est coutume de purifier les hydrolysats d'amidon.

Le sirop obtenu, parfaitement incolore et limpide, que l'on reconcentre à une matière sèche de 30 %, est alors prêt à subir l'étape de fractionnement chromatographique.

Séparation chromatographique

Le fractionnement de cet hydrolysat double acide-enzyme a été effectué dans l'installation chromatographique continue dont les détails de construction et de fonctionnement sont décrits dans le brevet américain US 4.422.881, ces détails n'étant repris ici que pour ce qui est nécessaire à la compréhension du procédé.

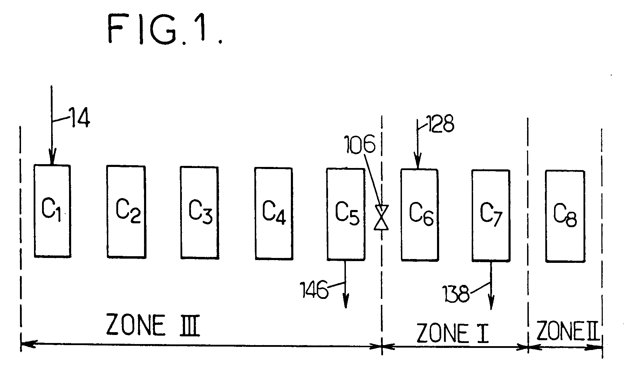

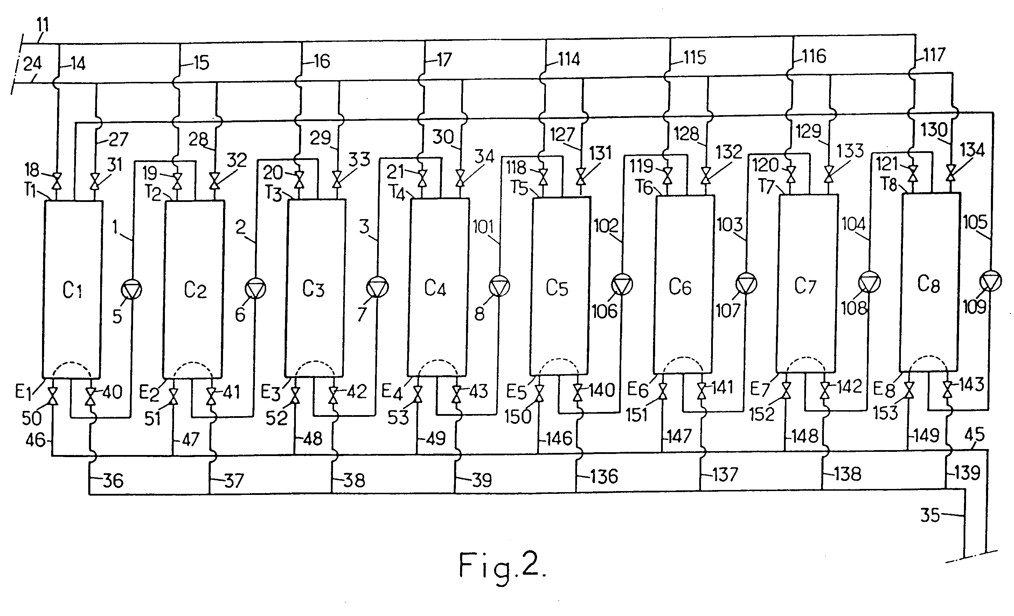

Cette installation comprend, comme montré à la figure 2 du brevet américain (reprise ici en tant que figure 1 et pour l'explication détaillée de laquelle on se référera audit brevet américain), huit colonnes ou étages $C_1$ à $C_8$ de 200 litres chacun, remplis de résine cationique forte macroporeuse C 150 commercialisée par la société PUROLITE, ces résines étant permutées sous la forme potassium, ayant une granulométrie comprise entre 400 et 1200 microns et un taux de réticulation de 7,5 %.

Par le calage des électrovannes, on établit dans cette installation une zone I de désorption de deux étages, correspondant aux colonnes 6 et 7, une zone II d'adsorption d'un seul étage correspondant à la colonne 8 et une zone III d'enrichissement et de séparation de l'hydrolysat d'amidon à faible indice de polymolécularité de 5 étages, correspondant aux colonnes 1 à 5, comme montré sur la figure 1. Cette figure 1 est une représentation schématique de l'installation selon la figure 2, mais on n'y a fait figurer que

- les colonnes $C_1$ à $C_8$,
- le dispositif de fermeture, en l'occurrence l'électrovanne 106,
- les canalisations d'alimentation en eau et en hydrolysat double, acide-enzyme à fractionner montrées respectivement en 128 et 14, et
- la canalisation 138 d'extraction du glucose et des polymères de glucose, de faible poids moléculaire retenus par la résine d'une part (fraction 2) et la canalisation 146 d'extraction de l'hydrolysat d'amidon à faible indice de polymolécularité (fraction 1).

Le dispositif de fermeture 106 maintient dans la configuration adoptée une étanchéïté totale entre, d'une part la zone III, qui est une zone d'enrichissement en polymères de haut poids moléculaire à l'extrémité de laquelle est récupéré l'hydrolysat d'amidon à faible indice de polymolécularité par la canalisation 146 et d'autre part la zone I de désorption du glucose et des polymères de glucose de faible poids moléculaire, zone en tête de laquelle est introduite l'eau de désorption.

Ce dispositif de fermeture 106 assure le sens du passage de la phase liquide sur la résine.

Une minuterie ajustée à 1500 secondes assure pour les débits suivants :

- Eau, par la canalisation 128, 366 litres/heure
- Hydrolysat d'amidon double acide-enzyme à 30 % de matières sèches, par la canalisation 14, 50 litres/heure
- Hydrolysat d'amidon à faible indice de polymolécularité, par la canalisation 146, 326 litres/heure.
- Glucose et polymères de glucose de faible poids moléculaire, par la canalisation 138, 90 litres/heure.

Au terme de 1500 secondes, toutes les entrées et sorties, de même que le dispositif de fermeture 106 sont décalés vers la droite.

Ces débits sont établis de telle manière qu'au terme des 1500 secondes la quantité d'hydrolysat d'amidon double, acide-enzyme soit compatible avec le volume de résine adsorbante et sa capacité d'adsorption. De même la quantité d'eau admise durant ce temps est celle qui permet de désorber la quasi-totalité du glucose et des polymères de faible poids moléculaire qui ont été adsorbés par la résine.

La part relative du rendement en polymères du glucose de faible indice de polymolécularité et des produits de faible poids moléculaire est assurée par le rapport des débits en sortie des canalisations 146 et 138. Dans les conditions retenues dans le cadre de cet exemple, le rendement pondéral en polymères de glucose de faible indice de polymolécularité est sensiblement égal à 50 % de la matière sèche introduite dans le système chromatographique.

Un accroissement de ce rendement, que l'on obtiendrait en augmentant le débit de la sortie 146 se traduirait par l'obtention d'un produit dont l'indice de polymolécularité serait accru et dont le Mp et le Mn seraient abaissés.

Une diminution de ce rendement aboutirait bien entendu au résultat inverse.

Pour effectuer cette chromatographie, la température à l'intérieur des colonnes est maintenue à 75°C.

La fraction X1, qui est composée en réalité dans ces conditions d'une partie d'eau pure provenant de l'élution de la colonne de résine du cycle précédent est scindée en deux sous-fractions. La première sous-fraction, représentant les 700 premières secondes du cycle de 1500 secondes, soit encore les 63,5 premiers litres de chaque cycle, est composée d'eau pratiquement pure qui n'est pas récupérée.

La seconde partie de cette fraction, qui s'écoule de l'installation par la sortie 146 entre la 701[e] seconde et la fin de chaque cycle de 1500 secondes est récupérée pour subir une purification supplémentaire. C'est cette fraction qui constitue à une matière sèche de 4,5 % environ l'hydrolysat d'amidon à faible indice de polymolécularité.

Cette fraction, qui n'est pas utilisable en l'état, doit subir pour son application en dialyse péritonéale continue et ambulatoire les traitements de purification qui suivent mais qui n'ont pas d'influence sur le Mp, le Mn ou l'indice de polymolécularité du produit obtenu après l'étape de chromatographie.

On peut bien entendu, faire précéder ces traitements de purification par les traitements d'hydrogénation catalytique ou d'isomérisation enzymatique qui ont déjà été décrits plus avant.

<u>Purification</u>

La deuxième partie de la fraction X1 issue de l'étape de chromatographie éventuellement hydrogénée ou isomérisée, est concentrée sous vide jusqu'à une matière sèche de 35 %. Elle est alors déminéralisée sur une batterie de résines échangeuses d'ions constituée d'une résine cationique forte permutée sous forme hydrogène, d'une résine anionique faible permutée sous forme hydroxyle et d'un lit mixte de résines cationique et anionique fortes également sous forme $H^+$ et $OH^-$. La déminéralisation est effectuée dans des conditions, de débit notamment, telles que l'on obtient une résistivité des sirops supérieure à $10^6$ ohms.cm.

A l'issue de la déminéralisation, le sirop est traité par 1 % de noir ACTICARBON 3S commercialisé par la société CECA afin d'éliminer les substances pyrogènes.

Ce sirop est ensuite filtré sur des filtres bactériologiques de maille 0,22 micron afin de le stériliser puis il est ensuite atomisé dans des conditions rigoureuses d'aseptie pour fournir une poudre parfaitement blanche, inodore et sans saveur.

Cet hydrolysat d'amidon à faible indice de polymolécularité révèle l'analyse suivante :

La teneur en DP + DP2 + DP3 est de 1 %, la teneur en polymères de glucose de poids moléculaire supérieur à 100.000 daltons est inférieure à 0,25 %.

Mp 14600
Mn 6300
Mp/Mn = 2,3

On remarquera l'indice de polymolécularité extrêmement faible du produit obtenu par le procédé selon l'invention.

A titre indicatif, l'analyse de la fraction $X_2$ rassemblant le glucose et les polymères de glucose de faible poids moléculaire a montré l'analyse suivante :

Mp 4400
Mn 870

L'hydrolysat d'amidon à faible indice de polymolécularité ainsi purifié et déshydraté est alors aisément remis en solution en compagnie d'autres électrolytes pour fournir des agents osmotiques extrêmement performants en dialyse péritonéale continue et ambulatoire.

**Revendications**

1. Procédé de fabrication d'un hydrolysat d'amidon présentant un indice de polymolécularité inférieur à 2,8 caractérisé par le fait qu'il consiste à :

   - hydrolyser par voie acide un lait d'amidon waxy jusqu'à un D.E. compris entre 8 et 15 ;
   - chromatographier l'hydrolysat ainsi obtenu sur résines cationiques fortes macroporeuses sous forme alcaline ou alcalino-terreuse ;
   - collecter l'hydrolysat d'amidon exclu lors de cette étape de chromatographie.

2. Procédé de fabrication selon la revendication 1, caractérisé par le fait que l'hydrolysat d'amidon obtenu présente un indice de polymolécularité inférieur à 2,5.

3. Procédé de fabrication selon l'une quelconque des revendications 1 et 2, caractérisé par le fait qu'après l'étape d'hydrolyse par voie acide, on hydrolyse par voie enzymatique l'hydrolysat acide, à l'aide d'$\alpha$-amylase bactérienne jusqu'à un D.E. compris entre 11 et 18.

4. Procédé de fabrication selon l'une quelconque des revendications 1 à 3, caractérisé par le fait que le rendement pondéral en hydrolysat d'amidon exclu est compris entre 10 et 80% de l'hydrolysat mis en oeuvre dans l'étape de chromatographie.

5. Procédé selon l'une quelconque des revendications 1 à 4, caractérisé par le fait que la chromatographie est effectuée sur des résines sous la forme potassium.

6. Procédé selon l'une quelconque des revendications 1 à 5, caractérisé en ce que l'hydrolysat d'amidon exclu est soumis à une étape de stabilisation thermique consistant en une hydrogénation catalytique ou en une isomération enzymatique transformant les extrémités réductrices en tréhalose.

7. Hydrolysat d'amidon cireux, complètement soluble dans l'eau et à faible indice de polymolécularité, caractérisé par le fait qu'il présente un indice de polymolécularité inférieur à 2,8, une masse moléculaire moyenne en poids Mp comprise entre 8.000 et 22.500 daltons et une masse moléculaire moyenne en nombre Mn inférieure à 8.000 daltons.

8. Hydrolysat d'amidon selon la revendication 7, caractérisé par le fait qu'il présente un indice de polymolécularité inférieur à 2,5.

9. Hydrolysat d'amidon selon l'une quelconque des revendications 7 et 8, caractérisé par le fait qu'il présente une masse moléculaire moyenne en poids Mp comprise entre 12.000 et 20.000 daltons.

10. Hydrolysat d'amidon selon l'une quelconque des revendications 7 à 9, caractérisé par le fait qu'il est hydrogéné ou isomérisé.

11. Hydrolysat d'amidon selon l'une quelconque des revendications 7 à 10, caractérisé par le fait qu'il contient moins de 3% de glucose et de polymères de glucose de DP inférieur ou égal à 3 et moins de 0,5% de polymères de glucose de DP supérieur à 600.

12. Application d'un hydrolysat d'amidon à faible indice de polymolécularité selon l'une quelconque des revendications 7 à 11 pour obtenir un agent osmotique destiné à être utilisé en dialyse péritonéale continue et ambulatoire.

## Claims

1. Process for the manufacture of a starch hydrolysate presenting a polymolecularity index lower than 2.8, characterized in that it consists in :

   - acid hydrolysis of a waxy starch milk up to a D.E. between 8 and 15 ;
   - chromatography of the hydrolysate thus obtained on macroporous strong cationic resins in alkali metal or alkaline-earth metal form ;
   - collection of the starch hydrolysate excluded during this chromatography step.

2. Process according to claim 1, characterized in that the starch hydrolysate obtained presents a polymolecularity index lower than 2.5.

3. Process according to any one of claims 1 and 2, characterized in that after the acid hydrolysis step, the acid hydrolysate is subjected to an enzymatic hydrolysis using bacterial $\alpha$-amylase, up to a D.E. between 11 and 18.

4. Manufacturing process according to any one of claims 1 to 3, characterized in that the weight yield of excluded starch hydrolysate is between 10 and 80% of the hydrolysate used in the chromatography step.

5. Process according to any one of claims 1 to 4, characterized in that the chromatography is carried out on resins in potassium form.

6. Process according to any one of claims 1 to 5, characterized in that the excluded starch hydrolysate is subjected to a thermic stabilization step consisting in a catalytic hydrogenation or in an enzymatic isomerization transforming the reducing ends into trehalose.

7. Waxy starch hydrolysate, completely water-soluble and of low polymolecularity index, characterized in that it has a polymolecularity index below 2.8, a weight-average molecular weight Mw between 8,000 and 22,500 daltons and a number-average molecular weight Mn below 8,000 daltons.

8. Starch hydrolysate according to claim 7, characterized in that it presents a polymolecularity index lower than 2.5.

9. Starch hydrolysate according to any one of claims 7 and 8, characterized in that it presents a weight-average molecular weight Mw between 12,000 and 20,000 daltons.

**10.** Starch hydrolysate according to any one of claims 7 to 9, characterized in that it is hydrogenated or isomerized.

**11.** Starch hydrolysate according to any one of claims 7 to 10, characterized in that it contains less than 3% of glucose and of glucose polymers of D.P. less than or equal to 3, and less than 0.5% of glucose polymers of D.P. greater than 600.

**12.** Use of a starch hydrolysate of low polymolecularity index according to any one of claims 7 to 11, in order to obtain an osmotic agent intended to be used in continuous and ambulatory peritoneal dialysis.

**Patentansprüche**

**1.** Verfahren zur Herstellung eines Stärkehydrolysats, das einen Polymolekularitätsindex kleiner als 2,8 aufweist, gekennzeichnet durch die Tatsache, daß es besteht aus:

- Hydrolysieren einer Wachsstärkemilch auf saurem Weg bis zu einem DE zwischen 8 und 15;
- Chromatographieren des so erhaltenen Hydrolysats über makroporöse starke kationische Harze in Alkali-oder Erdalkaliform;
- Gewinnen des bei diesem Chromatographieschritt ausgeschlossenen Stärkehydrolysats.

**2.** Herstellungsverfahren gemäß Anspruch 1, gekennzeichnet durch die Tatsache, daß das erhaltene Stärkehydrolysat einen Polymolekularitätsindex kleiner als 2,5 aufweist.

**3.** Herstellungsverfahren gemäß einem der Ansprüche 1 und 2, gekennzeichnet durch die Tatsache, daß man nach dem Schritt der Hydrolyse auf saurem Weg das saure Hydrolysat auf enzymatischem Weg mit Hilfe von bakterieller $\alpha$-Amylase bis zu einem DE zwischen 11 und 18 hydrolysiert.

**4.** Herstellungsverfahren gemäß einem der Ansprüche 1 bis 3, gekennzeichnet durch die Tatsache, daß die Gewichtsausbeute an ausgeschlossenem Stärkehydrolysat zwischen 10 und 80% des bei dem Chromatographieschritt verwendeten Stärkehydrolysats beträgt.

**5.** Verfahren gemäß einem der Ansprüche 1 bis 4, gekennzeichnet durch die Tatsache, daß die Chromatographie über Harzen in Kalium-Form ausgeführt wird.

**6.** Verfahren gemaß einem der Ansprüche 1 bis 5, dadurch gekennzeichnet, daß das ausgeschlossene Stärkehydrolysat einem Schritt zur thermischen Stabilisierung unterzogen wird, der aus einer katalytischen Hydrierung oder aus einer enzymatischen Isomerisierung, die die reduzierenden Enden in Trehalose überführt, besteht.

**7.** Wachsstärkehydrolysat, das in Wasser vollständig löslich ist und einen niedrigen Polymolekularitätsindex aufweist, gekennzeichnet durch die Tatsache, daß es einen Polymolekularitätsindex kleiner als 2,8, ein Gewichtsmittel des Molekulargewichts Mp zwischen 8.000 und 22.500 Dalton und ein Zahlenmittel des Molekulargewichts Mn kleiner als 8.000 Dalton aufweist.

**8.** Stärkehydrolysat gemäß Anspruch 7, gekennzeichnet durch die Tatsache, daß es einen Polymolekularitätsindex kleiner als 2,5 aufweist.

**9.** Stärkehydrolysat gemäß einem der Ansprüche 7 und 8, gekennzeichnet durch die Tatsache, daß es ein Gewichtsmittel des Molekulargewichts Mp zwischen 12.000 und 20.000 Dalton aufweist.

**10.** Stärkehydrolysat gemäß einem der Ansprüche 7 bis 9, gekennzeichnet durch die Tatsache, daß es hydriert oder isomerisiert ist.

**11.** Stärkehydrolysat gemäß einem der Ansprüche 7 bis 10, gekennzeichnet durch die Tatsache, daß es weniger als 3% Glucose und Glucosepolymere mit einem DP kleiner oder gleich 3 und weniger als 0,5% Glucosepolymere mit einem DP größer als 600 enthält.

**12.** Anwendung eines Stärkehydrolysats mit niedrigem Polymolekularitätsindex gemäß einem der Ansprüche 7 bis 11, um ein osmotisches Mittel zu erhalten, das zur Verwendung in der kontinuierlichen und ambulanten Peritonealdialyse bestimmt ist.

# FIG.1.

Fig.2.